Europäisches Patentamt

European Patent Office

Office européen des brevets

(19)

(11) Publication number: **0 073 210 B1**

# (12) EUROPEAN PATENT SPECIFICATION

(45) Date of publication of patent specification: **04.12.91**    (51) Int. Cl.⁵: **A61K 7/16, A61K 37/16**

(21) Application number: **82900611.3**

(22) Date of filing: **04.03.82**

(86) International application number:
**PCT/AU82/00022**

(87) International publication number:
**WO 82/03008 (16.09.82 82/22)**

(54) **CARIES INHIBITION.**

(30) Priority: **04.03.81 AU 7855/81**
           **23.06.81 AU 9429/81**

(43) Date of publication of application:
     **09.03.83 Bulletin 83/10**

(45) Publication of the grant of the patent:
     **04.12.91 Bulletin 91/49**

(84) Designated Contracting States:
     **AT CH DE FR GB LI NL SE**

(56) References cited:
     **GB-A- 825 115**
     **GB-A- 1 158 023**
     **US-A- 4 138 476**

(73) Proprietor: **THE UNIVERSITY OF MELBOURNE**
     **Grattan Street**
     **Parkville, Victoria 3052(AU)**

     Proprietor: **THE VICTORIAN DAIRY INDUSTRY**
     **AUTHORITY**
     **Domville Avenue**
     **Kew, Vic. 3101(AU)**

(72) Inventor: **REYNOLDS, Eric Charles**
     **The University of Melbourne Grattan Street**
     **Parkville, Vic 3052(AU)**
     Inventor: **STOREY, Elsdon**
     **The University of Melbourne Grattan Street**
     **Parkville, Vic 3052(AU)**
     Inventor: **MCDOUGALL, Wallace Arthur**
     **The University of Melbourne Grattan Street**
     **Parkville, Vic. 3052(AU)**

(74) Representative: **Baldock, Hugh Charles et al**
     **Lloyd Wise, Tregear & Co. Norman House**
     **105-109 Strand**
     **London, WC2R 0AE(GB)**

**Description**

This invention relates to compositions with caries- and gingivitis-inhibiting properties.

Galinski (GB-A-825115) has suggested the use of insoluble rennin casein for the inhibition of dental caries but discloses no tests to support this proposition. Huxley (Journal of Dental Research, Volume 56, No. 8, August 1977, Page 900) though postulating that casein may have cariostatic potential and setting out to test whether a diet containing casein might be less cariogenic than one containing lactalbumin, shows that the presence of casein did not affect the incidence of caries. Huxley also used the insoluble form of the protein - "casein". Non-soluble forms of casein are extremely unpalatable and for that reason are considered unsuitable as a caries and gingivitis inhibiting material for human use.

It is now found, however, that certain water-soluble caseins reduce the hydroxyapatite dissolution and substantially inhibit the adherence of the cariogenic bacterium to tooth material, which properties cannot be visualised from the aforesaid publications. As is shown hereinafter, these water-soluble caseins are soluble in the oral fluids where they are found to have a caries inhibitory and anti-gingivitis effect. Insoluble caseins do not have such effects.

Accordingly the invention provides an orally introducible composition comprising a casein compound and a carrier therefor which is either a foodstuff (including confectionery), a composition for topical application to teeth or to the mouth, or a pharmacologically acceptable carrier for oral administration, characterised in that said casein compound is a water-soluble casein which includes $\alpha_s$-casein and/or $\beta$-casein and is present in a caries- and gingivitis-inhibiting amount.

Preferably, the composition additionally contains urea.

The composition of this invention may be in the form of a foodstuff (including confectionery), dentrifice, tablet, lozenge or capsule, or comprise a pharmacologically acceptable carrier for topical application to the teeth or as a mouthwash.

Of particular interest as compositions are chewing gum, breakfast foods, ice-cream and other frozen confectionery, confectionery, sweets and cakes, as these are all known as caries-problem foods.

Also of particular interest are dentifrices, mouthwashes and preparations for topical application to teeth.

$\alpha_s$-casein and $\beta$-casein are obtainable from milk, and are present in water-soluble caseinates such as sodium caseinate and calcium caseinate.

TEST 1

The purpose of this test is to determine hydroxyapatite dissolution and in this respect since tooth enamel is largely composed of hydroxyapatite it is believed that useful comparisons can be made.

Double distilled, deionised water (greater than 10 MΩ/cm) was used throughout. Analytical reagent grade chemicals were obtained from Ajax Chemicals, Australia. Hydroxyapatite-spheroidal was purchased from BDH, and phosvitin from Sigma Chemical Co., Missouri, U.S.A. The milk proteins were fractionated by the method of Zittle and Custer (1) and their purity assessed by polyacrylamide gel electrophoresis using a modification of the method of Groves and Kiddy (2).

Methods

Hydroxyapatite Dissolution Rate Assay

A chromatography column (pharmacia K9/15) containing 1 g of hydroxyapatite beads was used for the demineralisation assay. Tris 5 mM, pH 8.3 containing 50 mM NaCl and 20 mg/l neomycin was used as the column influent buffer at 20°C and at a rate of 1.000±0.003 ml /min. A protein solution (10 mg 10 ml of influent buffer) was applied to the column and 1 ml fractions were collected before and after protein application and assayed for total calcium, phosphate and protein. From these values a rate of dissolution (nmol calcium or phosphate per min) for each 1 ml. fraction was obtained. Calcium, Phosphate and Protein Assays

Inorganic phosphate was measured by the method of Itaya and Ui(3) and protein by the method of Bradford (4). The determination of calcium was by atomic absorption spectrophotometry using 1% lanthanum chloride to prevent phosphate suppression.

Results

The proteins used for the study are listed in Table 1, they are all acidic proteins and included four phosphoproteins and three non-phosphorylated proteins from the whey fraction of bovine milk. The effect of the individual proteins on hydroxyapatite dissolution rate is shown in Table 2.

Table 1. Properties of various phosphorylated and non-phosphorylated acidic proteins.

| Protein | Molecular Weight | Phosphoserine Content [a] | Isoelectric Point | Carbohydrate Content |
|---------|------------------|---------------------------|-------------------|----------------------|
| Phosvitin | 35,500 | 110 | 1.5 | + |
| $\alpha_s$-casein | 23,613 | 8 | 4.1 | - |
| $\beta$-casein | 24,020 | 5 | 4.5 | - |
| $\kappa$-casein | 19,037 | 1 | 3.7 | + |
| $\alpha$-lactalbumin | 14,174 | - | 5.1 | - |
| $\beta$-lactaglobulin | 18,362 | - | 5.3 | - |
| Bovine serum albumin | 66,210 | - | 4.7 | - |

a. Residues per mol protein

Table 2

| Effect of phosphorylated and non-phosphorylated proteins on hydroxyapatite dissolution rate. | | | |
|---|---|---|---|
| Protein | Reduction in calcium dissolution rate[a] (nmol/min) | Reduction in phosphate dissolution rate (nmol/min) | Amount of protein bound (mg) |
| Phosvitin | 93.1± 5.4[b] | 63.8± 9.4 | 1.87±0.62 |
| $\alpha_s$-casein | 100.1± 4.1[b] | 63.5± 3.3 | 5.58±0.03 |
| $\beta$-casein | 94.8± 11.7[b] | 64.0± 19.3 | 7.45±0.37 |
| $x$-casein | 56.3± 8.9 | 33.7± 6.8 | 4.17±0.26 |
| $\alpha$-lactalbumin | 2.7± 1.7 | 2.9± 0.6 | 0.48±0.17 |
| $\beta$-lactoglobulin | 17.1± 1.7 | 12.5± 1.2 | 1.80±0.71 |
| Bovine serum albumin | 31.6± 4.5 | 20.5± 3.3 | 2.09±0.05 |

a. means±SD, n = 3
b. not significantly different P>0.5

In a trial of the above test the dissolution rate of hydroxyapatite as measured by the rate of calcium and phosphate released from the hydroxyapatite column was constant over a two hour period calcium 353.6±3.9 nmol/min, phosphate 225.4±6.8 nmol/min. The dissolution rates obtained using different hydroxyapatite columns showed greater variation, calcium 354.2±23.8 nmol/min, phosphate 229.6±30.8 nmol/min, n = 11. This intercolumn variation in dissolution rate could be attributable to different column packing resulting in a different HA surface area exposed.

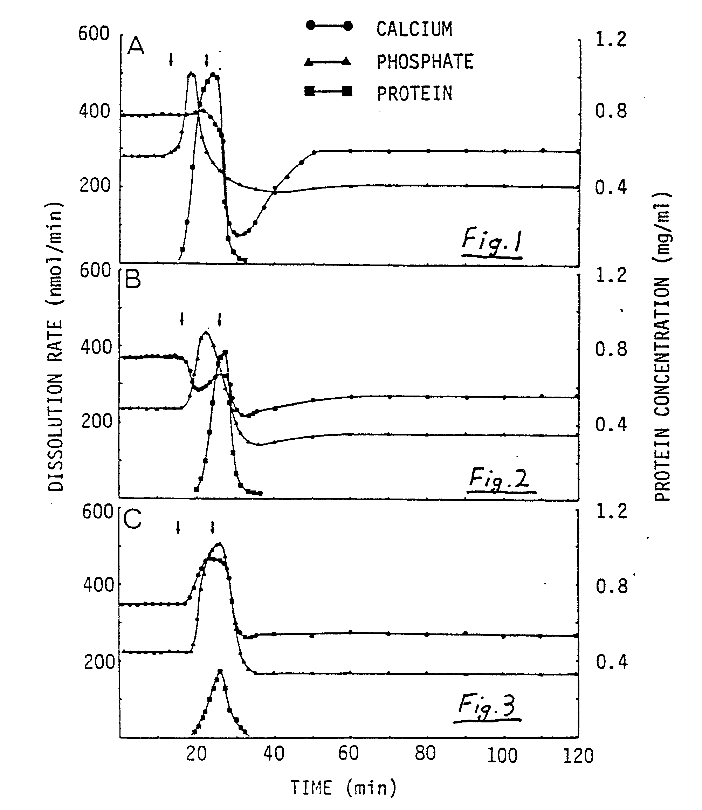

The effect of different materials on the dissolution rate of hydroxyapatite is shown in Figure 1. For example, phosvitin caused an initial increase in the dissolution rate of phosphate which then decreased and approached a new steady state level; 63.8 nmol/min less than the rate prior to phosvitin application. The protein caused an immediate and marked drop in the calcium dissolution rate which then increased and approached a steady-state level 93.1 nmol/min less than that prior to phosvitin application. The amount of protein that passed through the column was measured and from this the amount retained was calculated 1.87 mg. The dissolution rate returned to the original value after phosvitin was eluted from the column with 10 ml of eluent buffer containing 1.5 M phosphate, followed by buffer not containing phosphate.

The trace for $\alpha_s$-casein was very similar to that of phosvitin except that the immediate drop in calcium dissolution rate was not as marked Fig. 2. The difference in the steady-state rates of calcium and phosphate released before and after $\alpha_s$-casein application were very similar to those in Figure 1 (calcium, 100.1 nmol/min. phosphate 63.5 nmol/min).

The results obtained for $\beta$-casein (Fig. 3) were characteristic of all the other proteins tested, except for the final steady-state rates of calcium and phosphate released. All proteins (with the exception of phosvitin and $\alpha_s$-casein) caused an initial increase in the dissolution rate of both calcium and phosphate which then decreased as the proteins passed out of the column. The mean differences between tie steady-state dissolution rates before and after protein application, together with the amount of protein bound, for all proteins tested is presented in Table 2 above. The results show that the four phosphoproteins gave a marked reduction in the steady-state dissolution rate of HA with $\alpha_s$-casein and $\beta$-casein giving the same reduction in calcium and phosphate dissolution.

The tests have shown that all the acidic proteins caused a reduction in the steady-state dissolution rate of hydroxyapatite. However, the greatest reduction was given by $\alpha_s$-casein and $\beta$-casein. Some reduction was also given by $x$-casein, which is also present in water-soluble caseinates such as calcium and sodium caseinates.

From the above and from other data which suggests that adjacent phosphate groups of polyphosphoserine compounds have a spacing of about $6.88 \times 10^{-1}$ nanometers (6.88 Å) when in a beta-pleated sheet configuration and that calcium atoms in a hydroxyapatite surface along the c-axis will also be spaced at about $6.88 \times 10^{-1}$ nanometers, we speculate that a phosphate group-calcium atom bond materially reduces hydroxyapatite dissolution.

References:

1. Zittle, C.A., Custer, J.H.: Purification and some properties of $\alpha_s$-casein, J. Dairy Sci 46: 1183-1189, 1963.
2. Groves, M.L., Kiddy, C.A.: Polymorphism of $\gamma$-casein in cow's milk. Arch. Biochem. Biophys 126: 188-193, 1968.
3. Itaya, K., Ui, M.: A new micromethod for the colorimetric determination of inorganic phosphate. Clin, Chim, Acta 14: 361-366, 1966.
4. Bradford, MM.: A rapid and sensitive method for the quantitation of microgram quantities of protein utilizing the principle of protein-dye binding. Anal. Biochem. 72:248-254, 1976.

## TEST 2

For the purpose of in vivo testing, the following test procedure for determining the effect of casein and whey protein on caries incidence in the Sprague-Dawley rat.

Materials and Methods.

Forty-five, weanling, male Sprague-Dawley rats, 18 days old, bred from animals fed a fluoride free diet were used. The rats were marked for identification and then randomly distributed with respect to diet. They were housed in raised-bottom stainless-steel cages, one group of 15 per cage and fed a powdered cariogenic diet with either deionised water (control), a 2% casein solution or a 2% whey protein solution ad libitum. The cariogenic diet was a modified MIT - 200 diet shown in Table 3.

Table 3

| Composition of modified MIT - 200 cariogenic diet | |
| --- | --- |
| Component | % wt |
| Sucrose [a] | 67 |
| Wheyprotein Concentrate | 20 |
| Salt mixture [b] | 3 |
| Cottonseed oil | 3 |
| Cellulose [a] | 6 |
| Vitamin mixture [a] [b] | 1 |

a. Calcium and phosphate not detectable, fluoride content of complete diet was less than 0.2 g per g dry weight.
b. Vitamin and Salt mixture described in detail elsewhere.

The animals were weighed daily and the amounts of powdered diet and liquid consumed over a 24 h period by each group was measured. After 42 days on the diet, the animals were killed by cervical dislocation and treated as described below. Caries evaluation

Fissure caries was assessed using the method of Konig, Marthaler and Muhlemann (1958)(5). The mandible was removed from each rat and placed in formol-saline. The jaws were then sectioned and stained by the method of Konig et al (1958)(5), as described by Green and Hartles (1966)(6) to provide series of 100 $\mu$m thick longitudinal mesio-distal sections of the molar teeth. Only the main fissures of the first and second molar teeth were assessed for caries.

Results

Diet Consumption

The relative consumption of solid and liquid diet by the three groups of rats was tested by an analysis of variance (by diet). This showed that the quanitites of both solid and liquid consumed by each group were not significantly different ($P > 0.75$).

Caries Analysis.

The caries data shown in Table 4 were analysed in an analysis of variance table by diet.

Table 4

| Caries Experience Data | |
| --- | --- |
| Animals | Number of carious fissures [a] |
| Control Group | 7.92±2.06 |
| Casein Group | 1.87±2.50 |
| Whey Protein Group | 4.73±3.85 |

a. Maximum number possible 10.

The animals drinking the 2% casein solution had 76.5% less carious fissures than the control animals ($p < 0.001$), and the animals consuming the 2% whey protein solution had 40.3% less carious fissures than the control group ($p < 0.01$). The correlation of caries incidence with the final weight of the rat was tested for the three groups. No correlation attained significance ($p > 0.1$).

Similarly, the initial and final weights showed no correlation, nor were weight gain and caries incidence correlated.

Conclusion

Acidic proteins in the drinking water substantially reduced caries incidence of male Sprague-Dawley rats, however the phosphorylated protein (casein) caused a greater reduction than the non-phosphorylated whey proteins.

References:

(5) Konig K.G., Marthaler T.M. and Muhlemann H.R. 1958: Methodik der Kurzfristig erzeugten Rattenkaries. Dr. Zahn-Mund-u. Kieferheilk. 29, 99-127.

(6) Green R.M. and Hartles R.L. 1966: The effect of differing high carbohydrates diets on dental caries in the albino rat. Br. J. Nutr. 20, 317-323.

TEST 3

This test was to determine the effect of protein on the adsorption of the bacterium Streptococcus mutans to hydroxyapatite.

Materials and Methods

Hydroxyapatite discs were prepared by pressing 150 mg of hydroxyapatite (Bio-Gel HTP, Biorad Laboratories) for 5 min in a KBr press under a pressure of $7.7 \times 10^7$ Pa (5 tons). The discs were hydrated then incubated with either various protein solutions or imidazole buffer (0.05M pH 7.0, containing 0.025 M NaCl). The adherence of $^3$H-labelled S.mutans PK1 cells was studied by incubating the pretreated discs with $^3$H-thymidine labelled cells ($10^9$ cells/ml) suspended in the imidazole buffer. The protein solutions used were all 5 mg/ml in imidazole buffer. The proteins and polypeptides studied were $\alpha_s$-casein, $\beta$-casein, x-casein, phosvitin, bovine serum albumin, histone III, histone VIII, $\alpha$-lactalbumin, $\beta$-lactoglobulin, poly-l-lysine and poly-l-glutamate. The caseins were prepared by selective precipitation and the other proteins were purchased from Sigma Chemical Co., Missouri, U.S.A.

Results

The effect of pretreating hydroxyapatite discs with various protein solutions on the adherence of S.mutans cells is shown in Table 5.

Table 5. Effect of protein on S. mutans adherence to hydroxyapatite.

| Proteins | Type | Number of S.mutans cells adsorbed ($\times 10^7$) |
|---|---|---|
| Control | | $1.9 \pm 0.6$[a] |
| $\alpha_{s1}$-casein | acidic phosphoprotein | $0.5 \pm 0.3$ |
| $\beta$-casein | acidic phosphoprotein | $0.4 \pm 0.4$ |
| $\kappa$-casein | acidic phosphoprotein | $0.6 \pm 0.1$ |
| phosvitin | acidic phosphoprotein | $0.5 \pm 0.1$ |
| BSA | acidic protein | $0.6 \pm 0.1$ |
| histone III | basic protein | $2.2 \pm 0.9$ |
| histone VIII | basic protein | $2.6 \pm 0.9$ |

6

| Proteins | Type | Number of S.mutans cells adsorbed $(\times 10^7)$ |
|----------|------|---------------------------------------------|
| -lactalbumin | acidic protein | $1.0 \pm 0.4$ |
| -lactoglobulin | acidic protein | $0.9 \pm 0.4$ |
| poly-lysine | basic polypeptide | $3.8 \pm 1.1$ |
| poly-glutamate | acidic polypeptide | $0.5 \pm 0.1$ |

a means $\pm$ SD, n=8

Conclusion

All the acidic proteins and polypeptides (especially the phosphoproteins) caused a reduction in bacterial adherence to hydroxyapatite. However, the basic proteins and polypeptides either had no effect or enhanced bacterial adherence to hydroxyapatite.

Having regard to the successful results obtained from using the above tests Applicants have formulated various compositions in accordance with this invention as as illustrated by the Examples which follow.

In general, the compositions of the Examples contain from 0.5-20% by weight of a water-soluble casein material which is either sodium caseinate or calcium caseinate. Sodium and calcium caseinate are the sodium and calcium salts, respectively, of whole casein which contains fractions of $\alpha_s$-casein, $\beta$-casein and kappa-casein.

Example 1. Flour: In a device for mixing dry substances, 1% by weight of powdered sodium caseinate was blended with flour.

Example 2. Cereal: A breakfast cereal was sprayed with a solution of calcium caseinate in water. The cereal flakes were then dried to produce a finished product containing 1% calcium caseinate.

Example 3. Bread: 2% by weight of calcium caseinate was added to the flour during the mixing of ingredients for the manufacture of bread.

Example 4. Cake mix: 1% by weight of calcium caseinate was added to the dry ingredients in the preparation of a cake mix.

Example 5. Confectionery: In the preparation of confectionery 2% by weight of calcium caseinate was added to the final mixture.

Example 5. Biscuit: In the preparation of a biscuit mixture 5% by weight of calcium caseinate was added to the other dry ingredients during mixing.

Example 7. Beverage: A beverage was prepared in which 1% weight of calcium caseinate had been dissolved.

Example 8. Tablet: A tablet was made containing 10% by weight of calcium caseinate together with excipients being flavouring matter and binding material.

In preparation of a typical dentifrice within the scope of this invention, the requisite salt and salts of the selected casein are incorporated into dentifrice compositions in any suitable manner depending on whether a powder, paste or liquid preparation is to be produced. For this purpose appropriate preparations of surface-active agents, binders, flavouring materials and other excipients required to achieve the required form of dentifrice are added.

The invention is further illustrated by the following examples: Example 9. Tooth paste: A toothpaste was prepared having the following composition:-

7

| Calcium caseinate | 5.0% by weight |
| Gum tragacanth | 1.0% " " |
| Saccharin | 0.1% " " |
| Glycerin (B.P.) | 20.0% " " |
| Sodium lauryl sulphate | 1.0% " " |
| Methyl parahydroxy benzoate | 0.1% " " |
| Flavouring and colouring | 1.0% " " |
| Dibasic calcium phosphate | 35.0% " " |
| Water | 36.8% " |

Example 10. Toothpaste: A preparation as set out in example 9 was repeated but with the addition of 2% sodium fluoride in a suitable form.

Example 11. Toothpaste: A preparation as set out in example 9 was repeated but with the addition of 0.4% stannous fluoride in a suitable form.

Example 12. Toothpaste: A preparation as set out in example 9 was repeated but with the addition of 0.1% mono sodium fluorophosphate in a suitable form.

Example 13. Tooth powder: The following preparation was made:-

| Calcium caseinate | 5.0% by weight |
| Soluble saccharin | 0.1% by weight |
| Colour Agent | Trace " " |
| Dibasic calcium phosphate | 94.1% by weight |

Example 14. Tooth powder: A preparation as set out in example 13 was made but with the addition of 1% monosodium fluorophosphate in a suitable form.

Example 15. Liquid dentifrice: A preparation was made consisting of:-

| Sodium alginate | 1.5% by weight |
| Calcium caseinate | 5.0% " " |
| Sodium lauryl sulphate | 1.0% " " |
| Flavouring | Trace " " |
| Colouring | Trace " " |
| Water | 92.5% " " |
| pH adjusted to 7.0 | |

Example 16. Liquid dentifrice: As for example 15 but with 0.5% sodium fluoride added.

Example 17. Mouthwash: The following preparation was made:-

8

```
Sodium caseinate        2.0% by weight
Sodium fluoride         0.5% "      "
Flavouring              Trace "      "
Colouring               Trace "      "
Water                   97.5% "      "
```

## Claims

1. An orally introducible composition comprising a casein compound and a carrier therefor which is either a foodstuff (including confectionery), a composition for topical application to teeth or to the mouth, or a pharmacologically acceptable carrier for oral administration, characterised in that said casein compound is a water-soluble casein which includes $\alpha_s$-casein and/or $\beta$-casein and is present in a caries- and gingivitis-inhibiting amount.

2. A composition as claimed in Claim 1, containing from 0.5 to 20% by weight of said water-soluble casein.

3. A composition as claimed in Claim 1 or Claim 2, wherein said water-soluble casein is sodium caseinate or calcium caseinate.

4. A composition as claimed in any preceding claim wherein said water-soluble casein is present in said composition in solution.

5. A composition as claimed in any preceding claim, in the form of a mouthwash, tablet, lozenge or capsule.

6. A composition as claimed in any one of Claims 1 to 4, in the form of a foodstuff (including confectionery).

7. A composition as claimed in any of Claims 1 to 4 in the form of a beverage.

8. A composition as claimed in any one of Claims 1 to 4 in the form of a dentrifice.

## Revendications

1. Composition à prise par voie orale, comprenant un composé à base de caséine et un support de celui-ci qui est soit un produit alimentaire (y compris des confiseries), une composition à usage topique pour les dents ou la bouche, soit un support pharmacologiquement acceptable en vue d'une administration orale, caractérisée en ce que ledit composé à base de caséine est une caséine hydrosoluble qui contient une $\alpha_s$-caséine et/ou une $\beta$-caséine, et qui est présent en quantité empêchant les caries et les gingivites.

2. Composition telle que revendiquée dans la revendication 1, contenant de 0,5 à 20 % en poids de ladite caséine hydrosoluble.

3. Composition telle que revendiquée dans l'une des revendications 1 ou 2, dans laquelle ladite caséine hydrosoluble est le caséinate de sodium ou le caséinate de calcium.

4. Composition telle que revendiquée dans l'une quelconque des revendications précédentes, dans laquelle la caséine hydrosoluble est présente en solution dans cette composition.

5. Composition telle que revendiquée dans l'une quelconque des revendications précédentes, sous forme de lavage buccal, de comprimé, de tablette à sucer ou de capsule.

6. Composition telle que revendiquée dans l'une des revendications 1 à 4, sous forme de produit alimentaire (y compris les confiseries).

7. Composition telle que revendiquée dans l'une des revendications 1 à 4, sous forme de boisson.

8. Composition telle que revendiquée dans l'une des revendications 1 à 4, sous forme de dentifrice.

**Patentansprüche**

1. Eine oral einnehmbare Zusammensetzung aus einer Kaseinkomponente und einem Trägerstoff, der entweder ein Nahrungsmittel (einschließlich Süßigkeiten), eine Zusammensetzung zur topischen Anwendung für Zähne oder Mund oder ein pharmakologisch annehmbarer Trägerstoff zur oralen Anwendung ist; in der genannten Kaseinkomponente ist ein wasserlösliches Kasein charakterisiert, das ein $\alpha s$-Kasein und/oder ein $\beta$-Kasein einschließt, und in einer Menge vorhanden ist, die Karies und Zahnfleischentzündung vorbeugt.

2. Eine Zusammensetzung wie unter 1.) beschrieben, die zwischen 0,5 und 20 Gewichtsprozent des genannten wasserlöslichen Kaseins enthält.

3. Eine Zusammensetzung wie unter 1.) oder 2.) beschrieben, in der das genannte wasserlösliche Kasein ein Natrium-Kasein oder ein Calcium-Kasein ist.

4. Eine Zusammensetzung wie in den oben genannten Ansprüchen, in der das genannte wasserlösliche Kasein in der genannten Zusammensetzung in einer Lösung vorliegt.

5. Eine Zusammensetzung wie in den oben angegebenen Beschreibungen, in Form einer Mundspülung, Tablette, Pastille oder Kapsel.

6. Eine Zusammensetzung wie unter 1.) bis 4.) beschrieben, in Form eines Nahrungsmittels (einschließlich Süßigkeiten).

7. Eine Zusammensetzung wie unter 1.) bis 4.) beschrieben, in Form eines Getränke.

8. Eine Zusammensetzung wie unter 1.) bis 4.) beschrieben, in Form einer Zahnpasta.

Fig.1

Fig.2

Fig.3